# EUROPEAN PATENT APPLICATION

(11) **EP 4 843 616 A1**
(43) Date of publication of application: **25.11.2026**
(21) Application number: 25178274.4
(22) Date of filing: 22.05.2025
(51) Int. Cl.: A61N 5/10

(54) **METHOD FOR PLANNING A RADIATION THERAPY**

(71) Applicant: Siemens Healthineers International AG, 6312 Steinhausen (CH)
(72) Inventor: Scheib, Stefan, 8820 Wädenswil (CH); Czeizler, Elena, 00390 Helsinki (FI)
(74) Representative: Müller Schupfner & Partner Patent- und Rechtsanwaltspartnerschaft mbB (Muc)

(57) **Abstract**

The invention relates to a computer-implemented method for supporting a planning of a radiation therapy on a patient anatomy, the method comprising the following steps:
- receiving input data, the input data comprising image data (11), optionally multi-modal image data, of the patient anatomy and a dose distribution (12) related to the patient anatomy and/or information from which the dose distribution (12) can be derived;
- applying a trained machine learning model, in particular a trained visual language model, on the input data to generate output data (30) that comprises information concerning the radiation therapy and being related to the input data.

## Description

The invention relates to a computer-implemented method for supporting a planning of a radiation therapy on a patient anatomy, a method for preparing radiation therapy plan parameters, a computer-implemented method for providing a trained machine learning model and a corresponding computer program, computer-readable storage medium and radiation therapy system.

Independent of the grammatical term usage, individuals with male, female or other gender identities are included within the term.

Radiation therapy planning is complex, labor intense and typically requires multi-disciplinary coordination in the department. Due to emerging new options in treatment techniques and plan generation approaches, the plan complexity is increasing even more, and plan qualification, ranking and comparison is becoming even more demanding. It is therefore quite challenging to review a single treatment plan because of the many manual steps and expertise required. Even if some information of the resultant treatment plan may be condensed in graphs and numbers such as DVHs (dose-volume histograms), technical plan view and complexity metrics (see, for example, Chiavassa S, Bessieres I, Edouard M, Mathot M, Moignier A. Complexity metrics for IMRT and VMAT plans: a review of current literature and applications. Br J Radiol 2019; 92: 20190270.), a manual review is still required, which is complex and time-consuming. For example, since DVHs are not able to give any spatial information of the dose distribution, it is often required to manually scroll through the three-dimensional (3D) dose distribution together with the patient anatomy and contours to assess the spatial characteristics of the 3D dose distribution. In case multiple plan alternatives exist, this process is even more time consuming to either pick the best suitable plan or to propose specific changes in the beam or dose optimization parameters to create a possible improved treatment plan with respect of the applied dose distribution. Furthermore, plan robustness and complexity need to be evaluated in order to ensure a robust dose application which is close to the planned dose application as currently only the planned dose distribution and not the delivered one is reported.

Typically, in the state of the art, there is no general workflow for radiation therapy planning and the planning may, for example, be discussed between a technician and a physician. There are some publications that propose plan quality metrics and evaluation criteria to define the general understanding of plan quality, see, for example, "What is plan quality in radiotherapy? The importance of evaluating dose metrics, complexity, and robustness of treatment plans", Hernandez, Victor et al., Radiotherapy and Oncology, Volume 153 (2020), 26-33. Such generalizations may help to increase the overall quality of treatment plans. However, the quality of the resulting treatment plans is still connected to the individual knowledge and experience of the responsible person, and the required effort and time for treatment planning is still high. Furthermore, different clinics might emphasize different aspects of a plan when they define the quality metrics for the generated plans. For example, in some clinics, it may be more important to have a homogeneous target coverage even if that leads to higher doses in certain organs at risk (OARs) located very close to or even partially overlapping the targets. On the other hand, in other clinics, a more heterogenous target coverage may be accepted if it leads to a better OAR protection.

It is therefore an object of the invention to provide a means to decrease the required effort in treatment planning. Furthermore, it is desirable to provide a means to decrease the dependence of the quality and safety of treatment planning on individual expertise and to decrease the chance of overlooking deficiencies in the treatment plan and/or the planned/applied dose distribution.

This object is met or exceeded by a method according to claim 1, a method according to claim 12, a method according to claim 13, a computer program or a computer-readable storage medium according to claim 14, and a radiation therapy system according to claim 15. Further advantages and features result from the dependent claims, the description and the attached figures.

In the following the solution according to the invention is described with respect to the claimed methods as well as with respect to the claimed system and computer program or computerreadably storage medium. Features, advantages or alternative embodiments herein can be assigned to the other claimed objects and vice versa. In other words, claims and embodiments for the system can be improved with features described or claimed in the context of the respective methods. In this case, the functional features of the method are implemented by physical units of the system.

According to a first aspect of the invention, a computer-implemented method for supporting a planning of a radiation therapy on a patient anatomy is provided. The method comprises the following steps:
- receiving input data, the input data comprising image data, optionally multi-modal image data, of the patient anatomy, and a dose distribution related to the patient anatomy and/or information from which the dose distribution can be derived;
- applying a trained machine learning model, in particular a trained visual language model, on the input data to generate output data that comprises information concerning the radiation therapy and being related to the input data.

Advantageously, the method according to the invention enables an automation of at least some aspects of the treatment planning workflow. In particular the invention allows a user to save time during the reviewing of a treatment plan by automatically providing information concerning the radiation therapy based on image data and a dose distribution. Furthermore, it can enable to improve the quality of the treatment plan in particular for inexperienced users. On the one hand, particular deficiencies in the quality of a plan and/or dose distribution can be revealed relatively quickly and efficiently. On the other hand, the absence of such deficiencies can be recognized quicker based on the output of the method. Hence, according to this method, the planning of the radiation therapy can efficiently be supported.

According to the method, input data is received. This input data may for example be received at least partially via a user input. The input data may also at least partially be received from another automated process. For example, a dose distribution may be calculated automatically, for example by a treatment planning algorithm.

Advantageously, the input data comprises image data of the patient anatomy. The image data may in particular be three-dimensional (3D) image data. The image may in particular comprise image data acquired by at least one medical imaging modality, such as computed tomography (CT), cone beam CT (CBCT), magnetic resonance imaging (MRI - also referred to as MRT, i.e. magnetic resonance tomography) and positron emission tomography (PET). The image data may in particular comprise anatomical image data. Advantageously, the image data may thus be taken in account in order to provide the information of the output data which may allow a particularly efficient and complete automatic analysis. The image data may optionally be multimodal image data. Generally, multi-modal image data refers to image data, that is acquired from different sources and/or in different ways. In particular, the image data may comprise image data acquired by different imaging modalities and/or from different operation modes of a modality. Multi-modal image data may be image data from different imaging modalities that is combined into one dataset. In particular, the image data may comprise functional and/or quantitative data acquired by an imaging modality, such as MRI, PET, CT and SPECT (singlephoton emission computed tomography), in addition to anatomic image data.

The input data further comprise a dose distribution that is related to the patient anatomy or corresponding information from which the dose distribution can be derived. A dose distribution generally describes the amount of radiation dose that is applied in radiation therapy. The dose distribution thus refers to the distribution of a dose on an object or subject, e.g. on a patient. The dose distribution is in particular a three-dimensional (3D) dose distribution. Advantageously, the dose distribution enables to take into account useful information such as dose gradients and enables the trained machine learning model to find information such as non-optimal gradients, hot spots or cold spots.

Additionally or alternatively, the input data may comprise information from which the dose distribution can be derived. The trained machine learning model or another applied model may be configured to derive the dose distribution from this information. For example, the trained model may be trained to generate a dose distribution, in particular three-dimensional dose distribution, from the image data and some additional information. The additional information may, for example, comprise a textual prompt and/or other treatment planning information such as the field geometry or energy level used for generating the desired 3D dose distribution. The trained model may be configured to trigger a call to another algorithm or trained model, such as a 3D dose prediction machine learning model, to generate a predicted dose distribution, in particular a predicted 3D dose distribution. The trained model may be configured to trigger a call to an automated treatment planning workflow in order to generate an optimized dose distribution. The trained model used for the task of generating the dose distribution may be a different model than the trained model used for the evaluation of the dose distributions to generate the output data. The different model may be a visual language generative model, in particular a multimodal visual language generative model. The information from which the dose distribution can be derived may comprise data regarding a treatment intent, a treatment type, preliminary technical plan parameters, and/or a prescription. Technical plan parameters may, for example, comprise settings for arcs, arc lengths, arc start and stop angles, monitor units per arc segment, static gantry ports. The technical plan parameters may be related to the anatomy of the patient. An arc describes a movement of an accelerator head of the radiation therapy system that is typically moving on a circular trajectory around the patient. The arc length may describe a length of a (partial) rotation of an arc. A "static gantry port" may describe the parameters of a treatment plan where the gantry (in particular comprising the accelerator head) is not arcing around a patient in a continuous fashion, but is fixed at one or several pre-planned angles and is irradiating the patient without gantry rotation. This may be referred to as the static gantry port. The leaves of a multi leaf collimator may be moving during dose application to create a pre-planned and optimized fluence pattern for the fixed beam entry or static gantry port.

The input data may, for example, be provided to the trained machine learning model as an image or a group of images. For example, different images from different modalities (such as CT, MRT, etc.) may be provided. Preferably, the image and the dose distribution or corresponding information may be provided via separate channels to the machine learning model. Optionally, some channels may be combined. For example, the dose distribution may be provided as an overlay on the image or the images of the group of images. The input data may be pre-processed, such as by applying a normalization, a scaling, a noise reduction or other pre-processing steps.

A trained machine learning model is applied on the input data. In general, a trained machine learning model can be understood to be an algorithm, that is adapted to learn from data, i.e. from training data, and generalize to other data. In particular, by training based on training data the machine learning model is able to adapt to new circumstances and to detect and extrapolate patterns. Another term for "trained machine learning model" is "trained function". In general, parameters of a machine learning model can be adapted by means of training. In particular, supervised training, semi-supervised training, unsupervised training, reinforcement learning and/or active learning can be used. Furthermore, representation learning (an alternative term is "feature learning") can be used. In particular, the parameters of the machine learning models can be adapted iteratively by several steps of training. In particular, within the training a certain cost function can be minimized. In particular, within the training of a neural network the backpropagation algorithm can be used. In particular, a machine learning model may comprise a neural network, a support vector machine, a decision tree and/or a Bayesian network, and/or the machine learning model may be based on k-means clustering, Q-learning, genetic algorithms and/or association rules. In particular, a neural network may be a deep neural network, a convolutional neural network or a convolutional deep neural network. Furthermore, a neural network may be an adversarial network, a deep adversarial network and/or a generative adversarial network.

The trained machine learning model may in particular comprise a trained visual language model (VLM), which may also be denoted as vision-language model. The visual language model may comprise a visual transformer architecture. Visual language Models (VLMs) are a class of generative multimodal models which can learn to process images and texts simultaneously in order to perform advanced vision-language tasks, such as visual question answering, image captioning, discussing about images, and others. Hence, VLMs can be used to analyze combinations of images and text, in order to generate output, e.g. a recommendation, based on the input. In the context of this invention, images may in particular comprise the input image data and, optionally, the dose distribution. Text data may comprise, for example, a treatment prescription, a treatment intent, a template, a cancer type, a cancer stage, a textual prompt, and/or preliminary plan parameters. The general concept and architecture of VLMs is known in the state of the art. For example, the concept of VLMs is described in Lin et al., arXiv:2312.07533v4. Model architectures that may be used for this invention are for example, VisualBERT as described in arXiv:1908.03557v1 [cs.CV], Flamingo as described in arXiv:2204.14198v2 [cs.CV], CLIP as described under https://openai.com/index/clip/, or the adapted VLM as described in arXiv:2405.10948v1 [cs.CV]. The VLM may comprise a vision encoder, that is configured to process input image data. The visual encoder may in particular be configured to extract visual features from image data. For example, the vision encoder may comprise a visual transformer architecture. The VLM may comprise an LLM model that is configured to generate output data based on the input data.

The output data advantageously comprises information concerning the radiation therapy and being related to the input data. In particular, the output data may be an image or an image plus text or only text. For example, the output data may comprise a statement concerning the radiation therapy. The output data may comprise an answer to a question that was put in among the input data. The output data may comprise at least one or several deficiencies in the quality of the radiation plan or the dose distribution or an affirmation that no deficiency exists. Advantageously, the output data may be arranged such that relevant information, such as specific technical plan parameters, metrics, or dose distribution features are shown directly in at least one anatomical image, in particular without the need for a user to scroll through multiple images to find deficiencies. The output data may comprise information about a type of deficiency, such as "hot spot", "cold spot", "dose spill", and/or "dose gradient". For example, the output data may comprise at least one text description of at least one image of the input data.

Advantageously, based on the output data, a user may identify or formulate local dose variations as part of a review. Optionally, the user may be given the opportunity via an input interface to input local dose variations which may then be used by the trained machine learning model or by a further trained machine learning model to generate modified parameter values for a plan optimization algorithm and trigger the generation of an improved dose distribution.

Optionally the output data may comprise a suggestion what is recommended to be changed, e.g. what plan parameter may be changed, and/or what could be changed to achieve a particular goal, such as increased protection for organs at risk or better coverage of a target by the dose distribution. A target generally specifies a target of the radiation therapy. The target may also be denoted as radiation therapy target. The target may in particular be a planning target or planning target volume (PTV).

According to an embodiment, the image data of the patient anatomy comprises segmented anatomy and/or at least one segmented target. The target may be a region of the body that is to be treated, for example a tumor. Segmented anatomy may, for example, comprise at least one segmented organ, in particular an organ at risk (OAR). The trained machine learning model may be configured to identify an anatomy and/or target in which a deficiency of the dose distribution lies. The output data may comprise an indication indicating the anatomy and/or target which has a deficit. Advantageously, with information comprising segmented anatomy and/or target the trained machine learning model may be configured to analyze at least one quantity dependent on the segmented anatomy and/or target, such as a dose gradient in an organ at risk or outside the tumor.

According to an embodiment, the input data further comprises at least one piece of text data concerning the radiation therapy. Advantageously, additional input can thus be considered when generating the output. For example, the at least one piece of text data may comprise a treatment prescription, a user question and/or preliminary plan parameters. For example, a user question may comprise a question such as "show me the location of hot and cold spots". The trained machine learning model may thus be trained to specifically generate output according to a user question. The at least one piece of text data may be received as text or may be received as speech data that is in particular transformed into text form. For example, an interface may be provided for a user to select text data, type in text data or input text data by speech. For example, preliminary plan parameters may be transmitted from an algorithm for generating plan parameters. In order to process text data, the trained machine learning model, in particular VLM, may comprise a textual encoder. The textual encoder may be configured to process textual input, tokenize it and generate textual embeddings. The trained machine learning model, in particular VLM, may comprise a multimodal connector, that is configured to combine, and optionally pool, extracted visual and textual embeddings. The trained machine learning model, in particular VLM, may be configured to process the combined visual and textual embeddings and generate the output data based on this input.

According to an embodiment, the input data further comprises clinical constraints and/or a therapy intent. Clinical constraints may, for example, comprise information about organs at risk and/or radiation dose constraints. The therapy intent may comprise a treatment prescription, a target, and/or a dose for the target. A target generally refers to a target area or target volume that is to be treated by the radiation therapy. The target may in particular be a targeted tumor. Advantageously, by inputting the clinical constraints and/or a therapy intent the trained machine learning model may be enabled to make a more precise assertion of the of the dose distribution and potential deficiencies.

According to an embodiment, the output data comprises output image data based on the input image data, the output image data comprising at least one marker, the at least one marker marking at least one location in the output image data that is relevant for the radiation therapy. The output image data may in particular comprise anatomical image data. The marker may be used to highlight the location in the output image data. Advantageously, the marker may thus enable to focus on the particular location. A location in the output image data that is relevant for the radiation therapy may, for example, be a dose distribution deficiency, such as a hot spot or a cold spot, or another artefact. A marker may, for example, be a circle around the location, such as a circle around a dose distribution deficiency, or a crosshair. In addition to the marker, a further annotation, such as a textual annotation, may be provided. Optionally, the output image data may further comprise the dose distribution, in particular the dose distribution overlaid on anatomical image data.

According to an embodiment, the information concerning the radiation therapy comprises at least one deficiency in the dose distribution that is provided when found by the trained machine learning model. Accordingly, the trained machine learning model is configured to find the at least one deficiency. A deficiency may in particular comprise one or several of: at least one hot spot, at least one cold spot, at least one dose gradient outside the target, in particular towards an organ at risk. Advantageously, this may allow to quickly and efficiently determine deficiencies by a user with the help of this method. The output data may comprise an information about a location, in particular anatomical location, of a deficiency. For example, the output data may comprise an image, e.g. anatomical image, showing a deficiency, such as a dose distribution deficiency, wherein the deficiency is in particular highlighted by a marker. For example, a deficiency, such as a hot spot, may be highlighted by a circle around the deficiency in an anatomical image. The output data may comprise information about the type of deficiency. Hence, the trained machine learning model may be configured to identify the type of deficiency.

According to an embodiment, the trained machine learning model is configured to find anatomical locations of relevant information and/or to determine additional relevant information. Relevant information is in particular to be understood such that it is relevant for the radiation therapy, preferably relevant information that is typically considered in a plan evaluation phase. The output data comprises at least one anatomical location of relevant information when found by the trained machine learning model. In particular the output data may comprise one or several of the following anatomical locations of relevant information: an anatomical location of at least one violated plan constraint, an anatomical location of at least one sub-optimal dose gradient, an anatomical location of at least one unwanted dose spill off, an anatomical location of at least one cold spot, in particular in the target, an anatomical location of at least one unwanted hot spot, in particular in at least one organ at risk and/or at least one target. Additionally or alternatively, the output data may in particular comprise following additional relevant information: a summary of the generated plan and dose, the summary in particular overviewing plan parameters, metrics and/or dose distributions features.

According to an embodiment, the trained machine learning model comprises multiple specialized sub-models, each trained for a different partial task, the multiple specialized sub-models in particular including one or several of the following: a model for identifying at least one location, a model for categorizing at least one deficiency, a model for summarizing a plan, a model for answering at least one input question. Advantageously, the sub-models may thus be trained specifically for their respective tasks allowing for a more efficient training, potentially requiring less training data for each task. The location to be identified may be any location, in particular anatomical location, as described above and generally herein. The deficiency may be a dose distribution deficiency, in particular as described above and generally herein. The plan to be summarized may in particular be a radiation treatment plan. For example, the radiation treatment plan may be summarized to give an overview to a user. The at least one input question may in particular be a user question input via a user interface. For example, a question may be "how high is the dose in this particular structure" or how high is the hot spot". Alternatively or additionally, a single model may be provided that is trained to perform multiple or all specialized partial tasks.

According to an embodiment, the input data comprises multiple preliminary radiation therapy plans and/or multiple dose distributions related to multiple preliminary radiation therapy plans, wherein the information of the output data comprises a comparison of the multiple dose distributions and/or the multiple preliminary radiation therapy plans, in particular allowing a user to rank the multiple dose distributions and/or the multiple preliminary radiation therapy plans based on the comparison. An input option may be provided for a user to provide the ranking and the input of the user may be received to determine the particular radiation therapy plan and/or dose distribution that is to be used for radiation therapy. Hence, advantageously, user input can be used to rank plans using the additional information of the anatomical location of dose deficiencies. This may be particularly advantageous, since dose optimization and calculation itself is already possible faster than previously and can be expected to speed up significantly in the future. Hence, the determination and comparison of multiple plans can be a valuable option to allow an optimization of the plan to be applied by selecting the most suitable one. With the proposed method according to this embodiment the optimal plan for a patient can be found quickly. The output data may further comprise a summary and/or an overview of each plan and/or dose feature corresponding to each plan. This may help a user that is planning the radiation therapy to get a better understanding whether there are different trade-offs being used in these alternative plans. Moreover, this could also help the user to make a more informed decision when choosing one plan and dose distribution (e.g., with one type of planning trade-off) over the other plans and dose distributions. Alternative planning trade-offs may be illustrated in the output data, e.g. via the dose distributions and their respective deficiencies.

According to an embodiment, the output data comprises a description and/or characterization of the dose distribution and its features. The description and/or characterization may comprise a text, a table and/or image data. For example, a text may be a description of a general dose level. For example, a table may be a table illustrating achieved values of clinical goals. Image data may include image data of anatomical regions that still contain problems, such as deficiencies. For example, based on this part of the output, a user feedback, such as a new request, may be input by a user.

According to an embodiment, the method comprises the following additional steps:
- giving a user an opportunity to give user feedback based on the output data;
- receiving the user feedback;
- generating modified algorithm parameters based on the user feedback for a plan optimizing algorithm;
- applying the plan optimizing algorithm based on the modified algorithm parameters, the plan optimizing algorithm being configured to adapt a preliminary radiation therapy plan the dose distribution is based on, such that an optimized radiation therapy plan is generated based on the user feedback. The feedback may specify the features and/or aspects of the preliminary radiation plan and/or of the dose distribution that should be improved. For example, the feedback may comprise a deficiency in the dose distribution that should be improved. For example, the feedback may specify to better protect a particular organ at risk from radiation exposure. The modified algorithm parameters may be generated by the trained machine learning model or by another algorithm. The algorithm may in particular be modified with respect to algorithm parameters that were used for generating the dose distribution of the input data. Hence, optionally, the original algorithm parameters may be received in order to modify them. For example, the plan optimizing algorithm may comprise a further trained machine learning model and/or a multi-criteria optimization (MCO) algorithm. The algorithm parameters to be modified and the corresponding modified algorithm parameters may be chosen such that a goal defined by the user feedback, in particular an improvement of a deficiency, is likely to be achieved. For example, the goal may be one or several of the following:
   - to locally edit the dose distribution to meet the plan constraints,
   - to locally edit the dose distribution to improve sub-optimal dose gradients,
   - to remove or change the dose spill off in unwanted regions,
   - to eliminate cold spots in the dose distribution,
   - to reduce hot spots in the dose distribution.

According to an embodiment, the method is performed for a dose distribution of an on-line adaptive radiation therapy plan. On-line adaptive radiation therapy plans are typically radiation therapy plans that have been revised or adapted based on an image of the day, which has been acquired at the start of a day's treatment session. Typically, a patient is treated in a fractionated manner, which usually means that 3 to 38 daily fractions, depending on tumor and treatment protocol are planned. In case major deviations of the anatomy are present, one may adapt the plan while the patient is on the table and treat the new plan, which is optimized to the anatomy of the day. This on-line or on-couch adaptation may be seen in contrast to the off-line, or offcouch adaptation, where the new image of the day is used to adapt the plan for one of the next treatment sessions. This is typically done in cases of tumor shrinkage or body weight changes of the patient, because the new plan is also valid for the next fraction. On-line adaptation is often used for anatomy changing daily. Advantageously, the method allows to perform a quick plan review and, optionally, ranking (such as original plan versus adapted plan) based on the (3D) dose distribution.

According to an embodiment, the output data is stored to be applied for future use. In particular the output data may be stored together with outcome data of the radiation therapy. The output data together with the outcome data may be used to draw conclusions for the future, in particular to improve a treatment plan for similar patients in the future.

According to a further aspect of the invention, a computer-implemented method for preparing radiation therapy plan parameters is provided. The method comprises the following steps:
- generating preliminary therapy plan parameters and a corresponding dose distribution;
- applying the method for supporting a planning of a radiation therapy on a patient anatomy as described herein;
- providing the output data to a user;
- providing the option for the user to give user feedback and receiving the user feedback;
- based on the user feedback, and in particular also based on the output data, generating a modified radiation therapy plan with radiation therapy plan parameters and a modified dose distribution.

All features and advantages of the method for supporting a planning of a radiation therapy on a patient anatomy may be adapted to the method for preparing radiation therapy plan parameters and vice versa. The image data that is used may optionally be acquired within the method itself. Hence, there may be an additional step of acquiring image data, in particular multimodal image data, of the patient by applying a medical imaging modality, such as computed tomography, cone beam CT, magnetic resonance imaging and positron emission tomography. Alternatively, image data, that is already acquired may be used. The steps of providing output data, receiving user feedback and generating a modified radiation therapy plan may be repeated at least once and/or multiple times, in particular such that an iterative process is applied. A radiation therapy plan may generally be understood such that it comprises machine instructions, in particular radiation therapy plan parameters, enabling a radiation therapy system to deliver a particular type of dose distribution. The radiation therapy plan typically comprises information such as collimator angle, how to rotate a treatment head, and a position and treatment angle.

According to a further aspect of the invention, a computer-implemented method for providing a trained machine learning model, in particular a machine learning model as described herein, the method comprising the following steps:
- receiving pairs of input training data and ground truth training data, the input training data comprising a set of image data of patient anatomy, at least one piece of text data concerning the radiation therapy, and a dose distribution related to the patient anatomy and/or a radiation therapy plan, and the ground truth training data comprising annotation information related to the corresponding input training data;
- training a machine learning model, in particular a visual language model, based on the input training data and the ground truth training data;
- providing the trained machine learning model.

Features, advantages or alternative embodiments of the method for providing a trained machine learning model can be assigned to the other claimed objects and vice versa. The training data may be based on large datasets of image data comprising patient anatomy image data and dose distribution, optionally also contours as well as corresponding text descriptions (such as annotations, treatment prescription, the resultant plan parameters) in such a database. Alternatively, image data may be supplemented by manually or automatically adding corresponding annotations for the training. Optionally, the machine learning model that is to be trained may be pretrained. There may be two training phases, namely a first pre-training phase and a second phase for fine-tuning. In the case of training a visual language model, the pre-training phase may aim to align visual feature space with the language model's textual features. The datasets used for the pre-training phase may contain pairs of images and text. During the pre-training phase, a multimodal connector module may be trained, while a vision encoder component and an LLM-backbone may be using some pre-trained components which remain frozen during this phase. During the fine-tuning phase it may in particular be aimed to learn to interpret and respond to the user's instructions. Datasets that are used for this phase may contain data points formatted as a triple, in particular the triple: question, answer, image. During fine-tuning, the multimodal connector and parts or the whole LLM model may be continued to be trained. Optionally, different, in particular specialized machine learning models, may be trained for different subtasks, e.g. one model may be trained to identify a location, one model for determining a category, one model to summarize the radiation therapy plan, another type of model to answers questions, e.g. "how high is the dose in this structure", how high is the hot spot", etc.

According to a further aspect of the invention, a computer program or a computer-readable storage medium, in particular non-transient storage medium, comprising instructions which, when the program is executed by a computing system, cause the computing system to carry out any of the methods as described herein. All features and advantages of the methods may be adapted to the computer program or storage medium and vice versa. The storage medium may be any computer-readable storage medium. For example, the storage medium may be an optical storage medium or a solid-state storage medium. The storage medium may, be a harddrive, a solid-state-disk a flash storage an online server etc.

According to a further aspect of the invention, a radiation therapy system comprising a computing system that is configured to carry out any one of the methods as described herein is provided. The computing system comprises an interface, configured for receiving input data and a processing unit that is configured to apply the trained machine learning model as described herein to the input data in order to generate the output data and/or that comprises a computer program as described herein. In particular, the machine learning model may be executed on a GPU (graphics processing unit). However, the machine learning model may also be executed on a CPU (central processing unit). The computing system or parts of it may be on site or remote.

The embodiments described herein may be combined with each other unless indicated otherwise.

The accompanying drawings illustrate various exemplary embodiments and methods of various aspects of the invention.
Fig. 1 shows a flow diagram of a computer-implemented method for supporting a planning of a radiation therapy on a patient anatomy according to an embodiment of the invention;
Fig. 2 shows a flow diagram of a computer-implemented method for preparing radiation therapy plan parameters according to an embodiment of the invention;
Fig. 3 shows a diagram of an exemplary architecture for a machine learning model, namely a visual language model that may be applied in the context of this invention;
Fig. 4 shows a flow diagram of a computer-implemented method for providing a trained machine learning model;
Fig. 5 shows image data for vertebra treatment together with a dose distribution both, in a top view (upper image) and in a side view (lower image); and
Fig. 6 shows a radiation therapy system according to an embodiment of the invention.

Similar elements are designated with the same reference signs in the drawings.

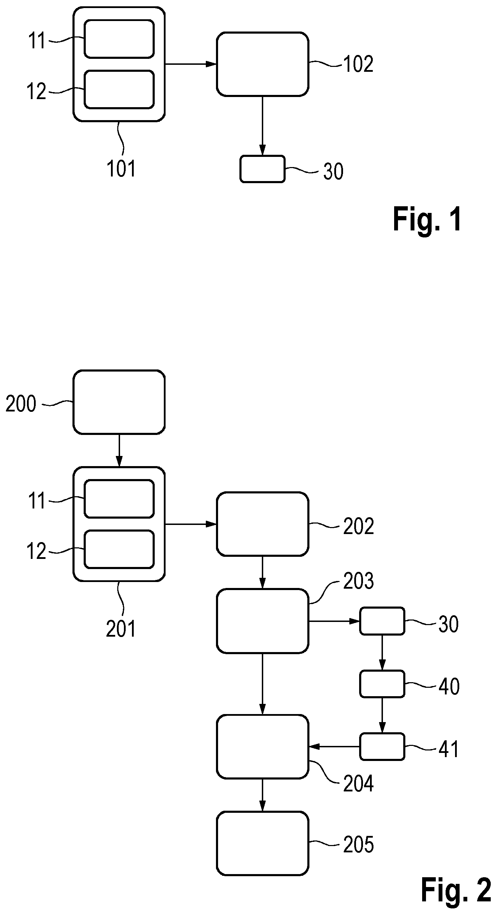

Figure 1 shows a flow diagram of a computer-implemented method for supporting a planning of a radiation therapy on a patient anatomy according to an embodiment of the invention. In a first step 101, input data is received. The input data comprises image data 11 of the patient anatomy. Optionally the image data 11 may be multi-modal image data. Preferably, the image data 11 may comprise segmented anatomy and/or at least one segmented target 71 of the radiation therapy. The input data further comprises a dose distribution 12 related to the patient anatomy or information from which this dose distribution 12 can be derived. Optionally, the input data may comprise further data. For example, the input data may further comprise at least one piece of text data concerning the radiation therapy, such as a treatment prescription, a user question and/or preliminary plan parameters. Optionally, the text data may comprise the user question, for example, "Are there any hot spots in this dose distribution?", or "Are there any cold regions within the target contours?", or "can you show me the slices where there is a hot spot within the structure Left_Parotid?" For example, the input data may further comprise clinical constraints and/or a therapy intent. In a further step 102, a trained machine learning model, in particular a trained visual language model, is applied on the input data to generate output data 30 that comprises information concerning the radiation therapy and being related to the input data. For example, the trained machine learning model may be configured to find deficiencies in the dose distribution 12. In this case, the information concerning the radiation therapy may comprise at least one deficiency in the dose distribution 12 that is provided when found by the trained machine learning model. A deficiency in particular comprises at least one hot spot 73, at least one cold spot 74, and/or at least one dose gradient outside the target 71, in particular towards an organ at risk 72. Optionally, the output data 30 may comprise output image data based on the input image data 11. In particular, the output image data may comprise at least one marker, the at least one marker marking at least one location in the output image data that is relevant for the radiation therapy. Optionally, the output data 30 may further comprise at least one anatomical location of relevant information, provided that such location was found by the trained machine learning model. Accordingly, the machine learning model may be configured to find such anatomical locations. The anatomical location may in particular be an anatomical location of at least one violated plan constraint, of at least one sub-optimal dose gradient, of at least one unwanted dose spill off, of at least one cold spot 74, in particular in the target 71, and/or of at least one unwanted hot spot 73, in particular in at least one organ at risk 72 and/or at least one target 71. Optionally, the output data 30 may comprise additional relevant information such as a summary of the generated plan and dose, the summary in particular overviewing plan parameters, metrics and/or dose distributions 12 features.

Figure 2 shows a flow diagram of a computer-implemented method for preparing radiation therapy plan parameters according to an embodiment of the invention. In a first step 200, preliminary therapy plan parameters and a corresponding dose distribution 12 are generated.

The following two steps 201, 202 and the output data 30 generated in these steps 201, 202 may correspond to the steps 101, 102 of the method described with respect to figure 1. In a first 201 of the following two steps 201, 202, input data is received by a trained machine learning model.

The input data comprises image data 11 of the patient anatomy, multi-modal image data. Furthermore, the received input data comprises the dose distribution 12 generated in step 200. Optionally, the input data may comprise further data, such as input data described with respect to figure 1. In a further step 202, the trained machine learning model, in particular a trained visual language model, is applied on the input data to generate output data 30 that comprises information concerning the radiation therapy and being related to the input data. Optionally, the output data 30 may comprise output data 30 as described with respect to figure 1. In a further step 203, the output data 30 is provided to a user 40. The user may examine the output data 30 and decide how to proceed further, in particular decide, whether changes in the preliminary radiation therapy plan should be applied due to the information from the output data 30. For this purpose, in a further step 204, the user is provided with the option to give user feedback 41 and the user feedback is received, e.g., via a user interface. In a further step 205, based on the user feedback, a modified radiation therapy plan with radiation therapy plan parameters and a modified dose distribution is generated.

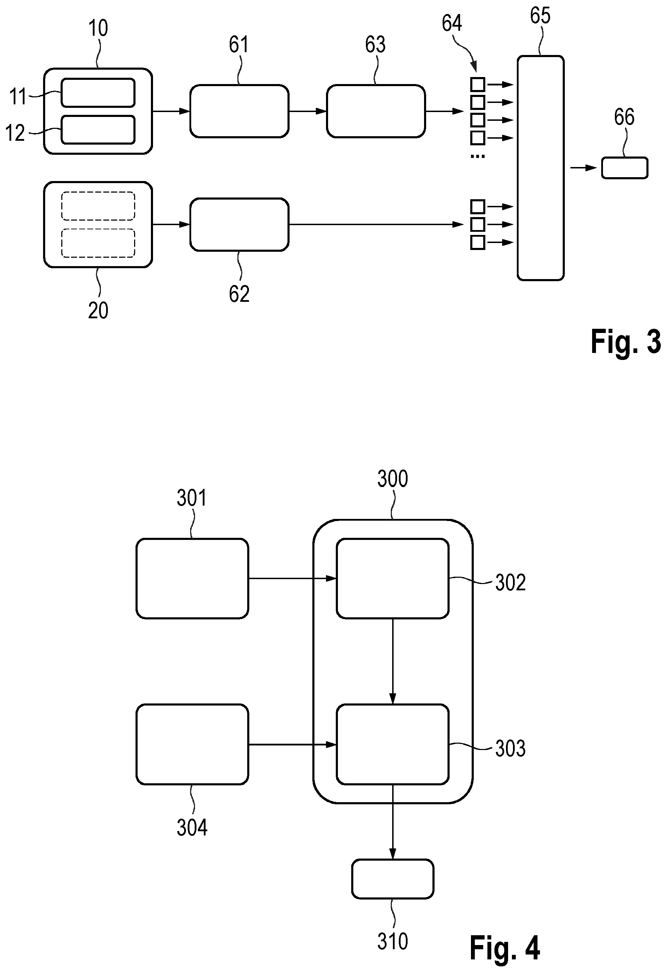

Figure 3 shows a diagram of an exemplary architecture for a machine learning model, namely a visual language model (VLM), that may be applied in the context of this invention. The VLM comprises a textual encoder 62, that is configured to process textual input 20, i.e. text data such as user questions, tokenize it and generate textual embeddings. The VLM further comprises a vision encoder 61, that is configured to process input images 10, comprising such as image data 11 of the patient anatomy and a dose distribution 12.. Additionally or alternatively, additional dose information and the request to generate a 3D dose distribution may be given as input text data in the textual encoder 20. The vision encoder 61 tokenizes input images, encodes them, extracts visual features from the input images, and generates visual embeddings. The vision encoder 61 may comprise a visual transformer architecture. The VLM further comprises a multimodal connector 63 that maps, and optionally also pools, the extracted visual embeddings to the same space as the textual embeddings. The tokens 64 shown in the image correspond to the actual embedding, which are also called "language tokens" or "image tokens". The VLM further comprises an LLM 65 (Large Language Model), that is configured to receive as input the combination of the visual and text embeddings and to generate the VLMs output 66.

Figure 4 shows a flow diagram of a computer-implemented method for providing a trained machine learning model. The machine learning model may, for example, have the architecture of the VLM as described with respect to figure 3. In this embodiment the training 300 comprises two phases, namely a pre-training phase in step 302 and an instruction fine-tuning phase in step 303. The pre-training phase aims to align visual feature space with the language model's textual features. This is in particular achieved by training the multimodal connector 63. For this purpose, training data comprising pairs of images and text are received (step 301) and used for the training. During the pre-training phase 302, the multimodal connector 63 is trained. The vision encoder component and the LLM may be using pre-trained components which remain frozen during this phase. During the instruction fine-tuning phase 303 the goal is to learn to interpret and respond to the input data, in particular also to the user's instructions. Training data used for this phase may comprise data points formatted as a triple, such as question, answer image. During fine-tuning, in particular the multimodal connector 63 and parts or the whole LLM are trained. In the end a trained machine learning model, in particular a trained VLM 310 is provided.

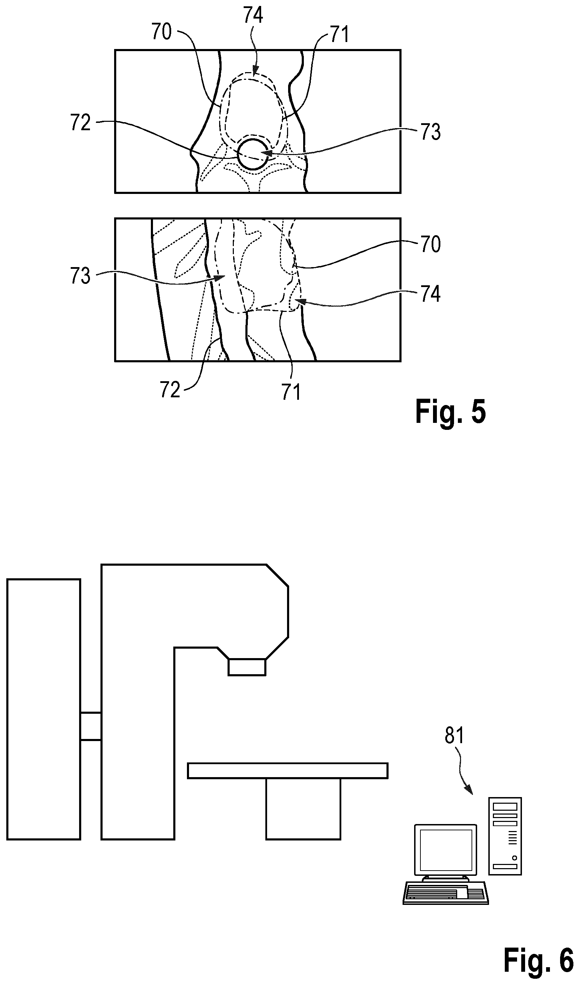

Figure 5 shows image data for vertebra treatment together with a visualization of a dose distribution 70 both, in a top view (upper image) and in a side view (lower image). The target 71 (also described as planning target volume - PTV) as well as an organ at risk 72 (in this example the spinal cord) are marked by contour lines. As can be seen, there is a cold spot 74 in the target 71, i.e. a volume where the dose distribution is lower than it should be, and a hot spot 73 in the organ at risk 72, namely a dose spill over into the organ at risk 72.

Figure 6 shows a radiation therapy system according to an embodiment of the invention. The radiation therapy system comprises a computing system 81 that comprises an interface configured for receiving input data and a processing unit configured to apply the trained machine learning model according to this invention, for example as described with respect to figures 1 to 3. The computing system 81 may optionally be a remote computing system.

### List of Reference

- 11: input image data
- 12: dose distribution
- 20: textual input
- 30: output data
- 40: user
- 41: user feedback
- 61: vision encoder
- 62: textual encoder
- 63: multimodal connector
- 64 65: LLM
- 66: output of the VLM
- 70: dose distribution
- 71: target
- 72: organ at risk
- 73: hot spot
- 74: cold spot
- 81: computing system

## Claims

1. A computer-implemented method for supporting a planning of a radiation therapy on a patient anatomy,
the method comprising the following steps:
- receiving input data, the input data comprising
> image data (11), optionally multi-modal image data, of the patient anatomy and
> a dose distribution (12) related to the patient anatomy and/or information from which the dose distribution (12) can be derived;
- applying a trained machine learning model, in particular a trained visual language model, on the input data to generate output data (30) that comprises information concerning the radiation therapy and being related to the input data.

2. The method according to claim 1,
wherein the image data (11) of the patient anatomy comprises segmented anatomy and/or at least one segmented target 71.

3. The method according to any one of the preceding claims,
wherein the input data further comprises at least one piece of text data concerning the radiation therapy.

4. The method according to any one of the preceding claims,
wherein the input data further comprises clinical constraints and/or a therapy intent.

5. The method according to any one of the preceding claims,
wherein the output data (30) comprises output image data based on the input image data (11), the output image data comprising at least one marker, the at least one marker marking at least one location in the output image data that is relevant for the radiation therapy.

6. The method according to any one of the preceding claims,
wherein the trained machine learning model is configured to find deficiencies in the dose distribution (12),
wherein the information concerning the radiation therapy comprises at least one deficiency in the dose distribution (12) that is provided when found by the trained machine learning model,
wherein a deficiency in particular comprises one or several of
- at least one hot spot (73),
- at least one cold spot (74),
- at least one dose gradient outside the target (71), in particular towards an organ at risk (72).

7. The method according to any one of the preceding claims,
wherein the trained machine learning model is configured to find anatomical locations of relevant information and/or to determine additional relevant information,
wherein the output data (30) comprises at least one anatomical location of relevant information when found by the trained machine learning model and/or additional relevant information, in particular one or several of the following:
- an anatomical location of at least one violated plan constraint,
- an anatomical location of at least one sub-optimal dose gradient,
- an anatomical location of at least one unwanted dose spill off,
- an anatomical location of at least one cold spot (74), in particular in the target (71),
- an anatomical location of at least one unwanted hot spot (73), in particular in at least one organ at risk (72) and/or at least one target (71),
- a summary of the generated plan and dose, the summary in particular overviewing plan parameters, metrics and/or dose distribution features.

8. The method according to any one of the preceding claims,
wherein the trained machine learning model comprises multiple specialized sub-models, each trained for a different partial task,
the multiple specialized sub-models in particular including one or several of the following: a model for identifying at least one location, a model for categorizing at least one deficiency, a model for summarizing a plan, a model for answering at least one input question.

9. The method according to any one of the preceding claims,
wherein the input data comprises multiple preliminary radiation therapy plans multiple dose distributions (12) related to multiple preliminary radiation therapy plans,
wherein the information of the output data (30) comprises a comparison of the multiple dose distributions (12) and/or the multiple preliminary radiation therapy plans, in particular allowing a user (40) to rank the multiple dose distributions (12) and/or the multiple preliminary radiation therapy plans based on the comparison.

10. The method according to any one of the preceding claims,
wherein the output data (30) comprises a description and/or characterization of the dose distribution (12) and its features.

11. The method according to any one of the preceding claims, the method comprising the following additional steps:
- giving a user (40) an opportunity to give user feedback (41) based on the output data (30);
- receiving the user feedback (41);
- generating modified algorithm parameters based on the user feedback (41) for a plan optimizing algorithm;
- applying the plan optimizing algorithm based on the modified algorithm parameters, the plan optimizing algorithm being configured to adapt a preliminary radiation therapy plan the dose distribution (12) is based on, such that an optimized radiation therapy plan is generated based on the user feedback (41).

12. A computer-implemented method for preparing radiation therapy plan parameters, the method comprising the following steps:
- generating preliminary therapy plan parameters and a corresponding dose distribution (12);
- applying the method according to any one of the preceding claims;
- providing the output data (30) to a user (40);
- providing the option for the user (40) to give user feedback (41) and receiving the user feedback (41);
- based on the user feedback (41), and in particular also based on the output data, generating a modified radiation therapy plan with radiation therapy plan parameters and a modified dose distribution.

13. A computer-implemented method for providing a trained machine learning model, in particular a machine learning model according to any one of the preceding claims, the method comprising the following steps:
- receiving pairs of input training data and ground truth training data,
the input training data comprising a set of image data (11) of patient anatomy,
at least one piece of text data concerning the radiation therapy, and
a dose distribution (12) related to the patient anatomy and/or a radiation therapy plan, and
the ground truth training data comprising annotation information related to the corresponding input training data;
- training a machine learning model, in particular a visual language model, based on the input training data and the ground truth training data;
- providing the trained machine learning model.

14. A computer program or a computer-readable storage medium, in particular non-transient storage medium, comprising instructions which, when the program is executed by a computing system (81), cause the computing system (81) to carry out the method according to any one of claims 1 to 13.

15. A radiation therapy system comprising a computing system that is configured to carry out the method according to any one of claims 1 to 12, the computing system (81) comprising
- an interface, configured for receiving input data and
- a processing unit that is configured to apply the trained machine learning model according to any one of claims 1 to 11 to the input data in order to generate the output data (30).
